# EUROPEAN PATENT SPECIFICATION

(11) **EP 0 724 436 B1**
(45) Date of publication and mention of the grant of the patent: **07.01.2004**
(21) Application number: 94929143.9
(22) Date of filing: 08.09.1994
(51) Int. Cl.: A61K 31/095, A61K 31/195, A61K 31/34, A61K 31/35, A61K 31/40, A61K 31/405, A61K 31/415, A61K 31/425, A61K 31/505, A61K 31/557, A61K 31/21, A61P 9/10

(54) **USE OF NITRIC OXIDE-ADDUCTS TO PREVENT THROMBOSIS ON ARTIFICIAL AND VASCULAR SURFACES**
VERWENDUNG VON STICKOXID-ADDUKTEN ZUR VERHÜTUNG VON THROMBOSEN AUF ARTIFIZIELLEN UND VASKULÄREN OBERFLÄCHEN
PRODUITS D'ADDITION DE L'OXYDE NITRIQUE UTILISES DANS LA PREVENTION DE LA THROMBOSE SUR DES SURFACES ARTIFICIELLES ET VASCULAIRES

(30) Priority: 17.09.1993 US 123331
(43) Date of publication of application: 07.08.1996
(73) Proprietor: BRIGHAM AND WOMEN'S HOSPITAL, Boston, Massachusetts 02115 (US); Nitromed, Inc., Bedford, MA 01730 (US)
(72) Inventor: FOLTS, John, D., Madison, WI 53705 (US); STAMLER, Jonathan, Chapel Hill, NC 27514 (US); LOSCALZO, Joseph, Dover, MA 02030-1812 (US)
(74) Representative: LOUIS- PÖHLAU- LOHRENTZ
(86) International application number: PCT/US1994/010027
(87) International publication number: WO 1995/007691

(56) References cited:
- CA-A- 2 106 105
- US-A- 4 734 097
- US-A- 4 900 719
- US-A- 5 002 964
- US-A- 5 004 461
- US-A- 5 025 001
- US-A- 5 071 649
- PAAPE ET AL.: "Leukocyte Adherance and plaque formation on polyethylene with altered surface properties" FEDERATION PROCEEDINGS, vol. 44, no. 3, 1985, page 449 XP002049691
- SIMON ET AL.: "Antiplatelet Properties of Protein S-Nitrosothiols Derived From Nitric Oxide and Endothelium-Derived Relaxing Factor" ARTERIOSCLER. THROMB., vol. 13, no. 6, 1993, pages 791-799, XP002049690
- HOLLMAN ET AL.: "Acute occlusion after percutaneous transluminal coronary angioplasty - a new approach" CIRCULATION, vol. 68, no. 4, 1983, pages 725-732, XP002050689
- NISHIMURA ET AL.: "Agents that modify EDRF formation alter antiplatelet properties of brain arteriolar endothelium in vivo" AM. J. PHYSIOL., vol. 261, no. 1 part 2, 1991, pages H15-H21, XP002049692
- SULLIVAN ET AL.: "Platelet Malondialdehyde in Cardiovascular Disease: Effect of Prostetic Heart Valves and Cardioactive Drugs on Production." THROMB. HAEMOSTASIS, vol. 44, no. 2, 1980, pages 76-80, XP002050690
- DEMBINSKA-KIEC ET AL.: "Selectin-P-mediated Adherence of Platelets to Neutrophils is Regulated by Prostanoids and Nitric Oxide" INT. J. TISS. REACT. , vol. 15, no. 2, 1993, pages 55-64, XP002049689
- DARIUS ET AL.: "The effects of Mosidomine and Its Metabolite SIN-1 on Coronary Vessel Tone, Platelet Aggregation, and Eicosanoid Formation In Vitro- Inhibition of 12-HPETE Biosynthesis" J. CARDIOVASC. PHARMACOL., vol. 6, no. 1, 1984, pages 115-121, XP002050691
- NISHIKAWA ET AL.: "Inhibition of Platelet Aggregation and Stimulation of Guanylate Cyclase by an Antianginal Agent Molsidomine and Its Metabolites" J. PHARMACOL. EXP. THER., vol. 220, no. 1, 1982, pages 183-190, XP002050692
- KOLANSKY ET AL.: "Immediate Postoperative Management" CARDIOVASC. CLIN., vol. 23, 1992, pages 277-291, XP002050693
- LOEB ET AL.: "Endothelium-dependent potentiation of human platelet aggregation" THROMB. RES., vol. 54, no. 5, 1989, pages 477-486, XP002049688
- IGNARRO ET AL.: "Endothelium-derived nitric oxide: actions and properties" FASEB JOURNAL, vol. 3, 1989, pages 31-36, XP002049693
- THROMBOSIS RESEARCH, Volume 26, No. 22, issued 1982, T. YAMAKADO et al., "Stimulation of Human Platelet Guanylah Cyclase by Nitroso Compounds", pages 135-140.
- BR. J. PHARMACOL., Volume 98, issued 1989, J. HENRY et al., "5-Nitrosothiols as Visodilators: Implications Regarding Tolerance to Nitric Oxide-Containing Vasodilators", pages 757-766.
- EUROPEAN JOURNAL OF PHARMACOLOGY, Volume 161, issued 1989, G. BUGA et al., "Endothelium-Derived Nitric Oxide Relaxes Non-Vascular Smooth Muscle", pages 61-72.

## Description

This invention relates to a method using nitric oxide adducts to prevent platelet deposition and thrombus formation on artificial surfaces which come into contact with blood. In addition, the invention relates to the use of nitric oxide adducts for the manufacture of a pharmaceutical preparation for applying directly to a damaged vascular surface in an animal to prevent platelet deposition or thrombus formation on the damaged surface. Typical nitric oxide adducts include nitroglycerin, sodium nitroprusside, S-nitroso-proteins, S-nitrosothiols, long carbon-chain lipophilic S-nitrosothiols, S-nitroso-dithiols, iron-nitrosyl compounds, thionitrates, thionitrites, sydnonimines, furoxans, organic nitrates, and nitrosated amino acids. The invention further relates to an artificial surface coated with a nitric oxide adduct.

Over the past two decades, much research effort has been directed towards the development of medical devices and machines that are used in a wide variety of clinical settings to maintain the vital physiological functions of a patient. For example, such devices as catheters, prosthetic heart valves, arteriovenous shunts, hemodialysis membranes, and cardiopulmonary bypass machines are used extensively in the treatment of cardiac and other diseases.

However, platelet deposition on artificial surfaces severely limits the clinical usefulness of such devices. Forbes, C.D., et al., Brit. Med. Bull. 34(2):201-207 (1978); Sheppeck, R.A., et al., Blood 78(3):673-680 (1991). For example, exposure of blood to artificial surfaces frequently leads to serious thromboembolic complications in patients with artificial heart valves, synthetic grafts and other prosthetic devices, and in patients undergoing external circulation, including cardiopulmonary bypass and hemodialysis. Salzman, E.W., Phil. Trans. R. Soc. Lond. B294:389-398 (1981).

The normal endothelium which lines blood vessels is uniquely and completely compatible with blood. Endothelial cells initiate metabolic processes, like the secretion of prostacyclin and endothelium-derived relaxing factor (EDRF), which actively discourage platelet deposition and thrombus formation in vessel walls. No material has been developed that matches the blood-compatible surface of the endothelium. In fact, in the presence of blood and plasma proteins, artificial surfaces are an ideal setting for platelet deposition (Salzman et al.). Exposure of blood to an artificial surface initiates reactions that lead to clotting or platelet adhesion and aggregation. Within seconds of blood contact, the artificial surface becomes coated with a layer of plasma proteins which serves as a new surface to which platelets readily adhere, become activated, and greatly accelerate thrombus formation (Forbes et al.).

This creates problems in the use of artificial materials at the microvascular level, where the ratio of vessel surface area to blood volume is high (Sheppeck, R.A., Blood 78(3):673-680 (1991)). For example, thromboembolism is still the most serious complication following prosthetic heart valve implantation, despite changes in design and materials used. In fact, the incidence of detectable thromboembolism can be as high as 50%, depending on the valve design and construction (Forbes et al.). Further, cardiopulmonary support systems used during cardiac surgery are responsible for many of the undesirable hemostatic consequences of such surgery (Bick, R.L., Semin. Thromb. Hemost. 3:59-82 (1976)). Thrombosis is also a significant problem in the use of prosthetic blood vessels, arteriovenous shunts, and intravenous or intraarterial catheters.

Conventional methods for preventing thrombus formation on artificial surfaces have a limited effect on the interaction between blood and artificial surfaces. For example, in cardiopulmonary bypass and hemodialysis heparin has little effect, and the only platelet reactions inhibited by anticoagulants are those induced by thrombin. In fact, it seems that heparin actually enhances the aggregation of platelets (Salzman, E.W., et al., J. Clin. Invest. 65:64 (1980)). To further complicate matters, heparin when given systemically, can accelerate hemorrhage, already a frequent complication of cardiac surgery.

Attempts to inhibit platelet deposit on artificial surfaces involve systemic administration of aspirin, dipyridamole, and sulfinpyrazone. While these have some effect in preventing thromboembolism when given with oral anticoagulants, serious adverse effects can result. Blood loss is significantly increased in bypass or hemodialysis patients following administration of aspirin (Torosian, M., et al., Ann. Intern. Med. 89:325-328 (1978)). In addition, the effect of aspirin and similarly acting drugs is not promptly reversible, which is essential during cardiopulmonary bypass. Finally, agents such as aspirin, which depress platelet function by inhibiting cyclooxygenase, may block platelet aggregation, but they do not prevent the adhesion of platelets to artificial surfaces (Salzman et al. (1981)).

Despite considerable efforts to develop non-thrombogenic materials, no synthetic material has been created that is free from this effect. In addition, the use of anticoagulant and platelet-inhibiting agents has been less than satisfactory in preventing adverse consequences resulting from the interaction between blood and artificial surfaces. Consequently, a significant need exists for the development of additional methods for preventing platelet deposition and thrombus formation on artificial surfaces.

In the same manner as artificial surfaces, damaged arterial surfaces within the vascular system are also highly susceptible to thrombus formation. The normal, undamaged endothelium prevents thrombus formation by secreting a number of protective substances, such as endothelium-derived relaxing factor (EDRF), which prevents blood clotting primarily by inhibiting the activity of platelets. Disease states such as atherosclerosis and hyperhomocysteinemia cause damage to the endothelial lining, resulting in vascular obstruction and a reduction in the substances necessary to inhibit blood clotting. Thus, abnormal platelet deposition resulting in thrombosis is much more likely to occur in vessels in which endothelial damage has occurred. While systemic agents have been used to prevent coagulation and inhibit platelet function, a need exists for a means by which a damaged vessel can be treated directly to prevent thrombus formation.

Therefore, in one aspect the invention relates to methods for preventing the deposit of platelets and for preventing thrombus formation on an artificial surface by contacting or coating the artificial surface with a nitric oxide adduct as specified in claim 1. Typical artificial surfaces, include catheters, prosthetic heart valves, artificial cardiopulmonary support machines, synthetic vessel graf ts, hemodialysis membranes, blood conduit tubing, blood storage bags, arteriovenous shunts, or artificial hearts.

Typical nitric oxide adducts include nitroglycerin, sodium nitroprusside, S-nitroso-proteins, S-nitrosothiols, long carbon-chain lipophilic S-nitrosothiols, S-nitroso-dithiols, iron-nitrosyl compounds, thionitrates, thionitrites, sydnonimines, furoxans, organic nitrates, and nitrosated amino acids.

Particularly preferred is the use of S-nitroso-proteins to coat thrombogenic surfaces. The use of such S-nitroso-proteins obviates the need for a polymer or other material to link the specific NO donor to the surface, such as a catheter wall. Rather, the catheter can be dipped or otherwise directly coated with solutions of the derivatized protein. Additionally devices which have been coated using S-nitroso-protein have the unique property that they can be dried and stored.

An additional particularly unique aspect of the invention is that this contemplates "recharging" the coating that is applied to a device, such as a catheter or other tubing as considered above, by infusing a nitric oxide donor to a previously coated surface. For example, an S-nitroso-protein such as S-nitroso albunium will lose its potency in vivo as the NO group is metabolized, leaving underivatized albumin. However, it has been recognized by the inventors that the surface coating can be "recharged" by infusing an NO donor such as nitroprusside. This principal is demonstrated by the experiments reported in Example 2 in which nitroprusside is mixed with albumin engendering subsequent protection against platelet deposition.

The invention also relates to a method for preventing the deposition of platelets on an artificial surface comprising coating the artificial surface with a nitric oxide adduct in combination with one or more anti-thrombogenic agents. Such agents include heparin, warfarin, hirudin and its analogs, aspirin, indomethacin, dipyridamole, prostacyclin, prostaglandin E₁, sulfinpyrazone, phenothiazines (such as chlorpromazine or trifluperazine) RGD (arginine-glycine-aspartic acid) peptide or RGD peptide mimetics, (See Nicholson et al., Thromb. Res., 62:567-578, 1991), agents that block platelet glycoprotein IIb-IIIa receptors (such as C-7E3), ticlopidine or the thienopyridine known as clopidogrel.

The invention further relates to an artificial surface coated with a nitric oxide adduct pursuant to claim 23.

The invention further relates to the use of nitric oxide adduct for the manufacture of a pharmaceutical preparation pursuant to any one of claims 11 to 13 for preventing thrombus formation on a damaged vascular surface in an animal. Coating of a damaged vascular wall to make it non-thrombogenic, as a mode of protection and/or treatment of endothelial tissue should be recognized as separate and distinct from the coating of an artificial surface, such as a synthetic polymer, with an NO donor.

Nonetheless the full concept of the invention disclosed herein is based on the discovery by the inventors that the use of nitric oxide adducts inhibits platelet deposition and thrombus formation on both artificial surfaces, and on damaged natural vascular surfaces.

Therefore, in one principal aspect the invention does comprise a method for preventing platelet deposition or thrombus formation on an artificial surface by treating the

artificial surface with a nitric oxide adduct. The term "artificial surface" refers to any synthetic material contained in a device or apparatus that is in contact with blood or blood products. Artificial surfaces may be used for both ex vivo or in vivo purposes. For example, artificial surfaces exist on intravenous or intraarterial catheters, prosthetic heart valves, synthetic blood vessel grafts, blood conduit tubing, or artificial hearts. Artificial surfaces are also contained in devices that are used to provide extracorporeal circulation for patients, such as cardiopulmonary bypass machines and charcoal or ion exchange resins or membranes used in hemodialysis.

By inhibiting platelet deposition on the artificial surface, treatment with a nitric oxide adduct also prevents thrombus formation. A thrombus is a pathologic blood clot formed in vivo within the heart or blood vessel. A thrombus may also form ex vivo under conditions of increased platelet adherence and activation, such as in tubing or blood storage bags. Thromboembolism occurs when a dislodged thrombus or part of a thrombus partially or completely occludes a blood vessel, and prevents oxygen transport to the affected tissues, ultimately resulting in tissue necrosis.

The term "nitric oxide adduct" refers to any compound that is capable of releasing a reactive nitric oxide species, such as NO⁻ (nitroxyl), NO⁺ (nitrosonium), and NO^{•} (uncharged NO radical). For example, nitric oxide adducts include low molecular weight S-nitrosothiols such as S-nitroso-N-acetylcysteine, S-nitroso-captopril, S-nitroso-homocysteine, S-nitroso-cysteine, S-nitroso-glutathione, and S-nitrosopenicillamine. S-nitrosothiols and the methods for preparing them are described in U.S. Patent Application No. 07/943,834, filed September 14, 1992, Oae, et al., Org. Prep. Proc. Int., 15(3):165-198 (1983); Loscalzo et al., J. Pharmacol. Exp. Ther., 249(3):726729 (1989) and Kowaluk et al., J. Pharmacol. Exp. Ther., 256:1256-1264 (1990), all of which are incorporated in their entirety by reference.

Other suitable nitric oxide adducts include S-nitroso-angiotensin converting enzyme inhibitors (hereinafter referred to as S-nitroso-ACE inhibitors) which are described in Loscalzo, U.S. Patent No. 5,002,964 (1991) and Loscalzo et al., U.S. Patent No. 5,025,001 (1991) both of which are incorporated in their entirety by reference. Examples of such S-nitroso-ACE inhibitors include compounds having the formula: 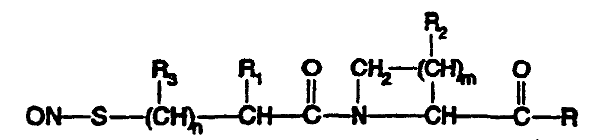 wherein
R is hydroxy, NH₂, NHR⁴, NR⁴R⁵, or C₁-C₇ alkoxy, wherein R⁴ and R⁵ are C₁-C₄ alkyl, or phenyl, or C₁-C₄ alkyl substituted by phenyl;
R¹ is hydrogen, C₁-C₇ alkyl, or C₁-C₇ alkyl substituted by phenyl, amino, guanidino, NHR⁶, NR⁶R⁷, wherein R⁶ and R⁷ are methyl or C₁-C₄ alkanoyl;
R² is hydrogen, hydroxy, C₁-C₄ alkoxy, phenoxy, or C₁-C₇ alkyl;
R³ is hydrogen, C₁-C₄ or C₁-C₇ alkyl substituted by phenyl; m is 1 to 3; and
n is 0 to 2.

Other suitable S-nitroso-ACE inhibitors include N-acetyl-S-nitroso-D-cysteinyl-L-proline, N-acetyl-S-nitroso-D,L-cysteinyl-L-proline, 1-(4-amino-2-S-nitroso)mercaptomethylbutanoyl)-L-proline, 1-[2-hexanoyl]-L-proline, 1-[5-guanidino-2-(S-nitroso)mercaptomethyl-pentanoyl]-L-proline, 1-[5-amino-2-(S-nitroso) mercaptomethyl-pentanoyl]-4-hydroxy-L-proline, 1-[5-guanidino-2-(S-nitroso)mercaptomethyl-pentanoyl]-4-hydroxy-L-proline, 1-[2-aminomethyl-3(S-nitroso)-mercaptomethyl-pentanoyl-L-proline, and S-nitroso-L-cysteinyl-L-proline.

Additional suitable S-nitroso-ACE inhibitors include those having the following formulas: 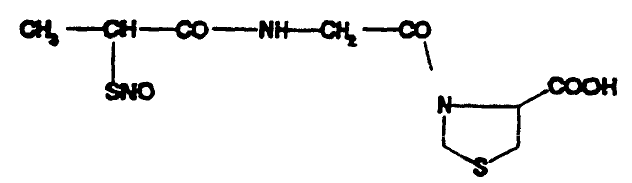 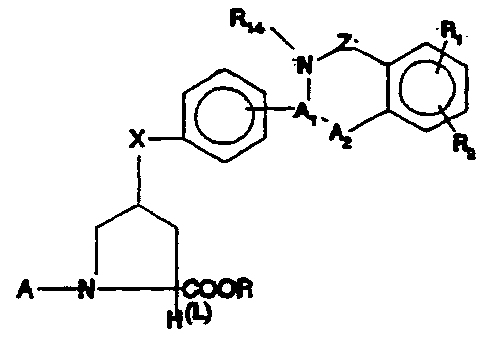 wherein
X is oxygen or sulfur;
-A₁,-A₂- is CH-NH or -C=N-;
A is 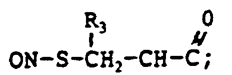
R is selected from hydrogen, lower (C₁-C₄) alkyl, benzyl, benzhydryl, and salt forming ion;
R₁ and R₂ are independently selected from hydrogen, halogen, lower alkyl, lower alkoxy, halo substituted lower alkyl, nitro, and SO₂NH₂;
Z is 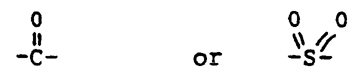
R₃ is hydrogen, lower alkyl, halo substituted lower alkyl, phenyl, benzyl, phenethyl, or cycloalkyl; and
R₄ is hydrogen, lower alkyl, halo substituted lower alkyl, hydroxy substituted lower alkyl, -(CH₂)_{q}-N (lower alkyl)₂ or -(CH₂)_{q}-NH₂ and q is one, two, three or four.

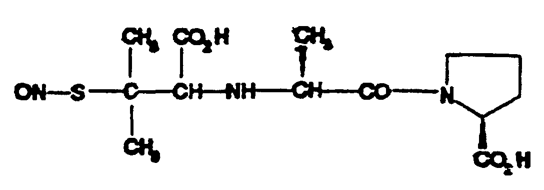

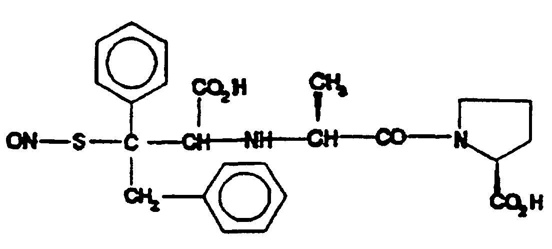

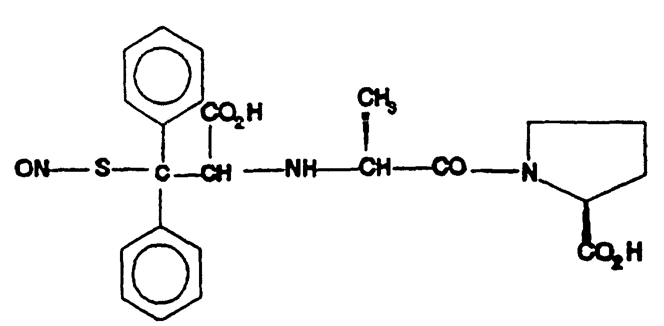

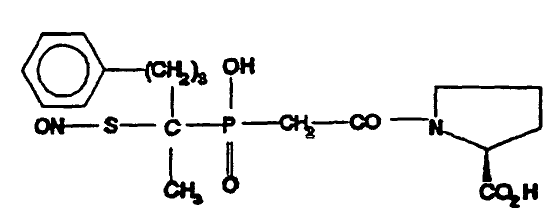

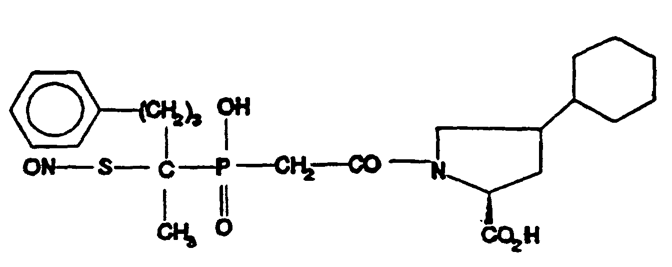

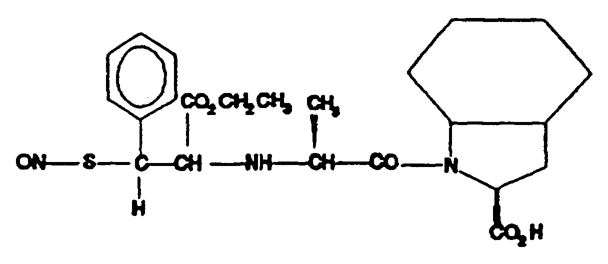

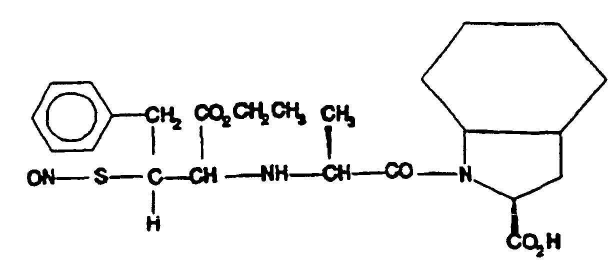

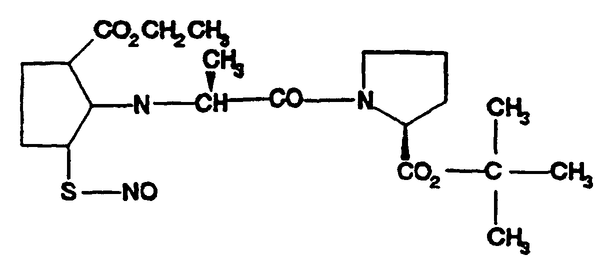

The S-nitroso-ACE inhibitors can be prepared by various methods of synthesis. In general, the thiol precursor is prepared first, then converted to the S-nitrosothiol derivative by nitrosation of the thiol group with NaNO₂ under acidic conditions (pH = 1 to 5) which yields the S-nitroso derivative. Acids which may be used for this purpose include aqueous sulfuric, acetic and hydrochloric acids. Thiol precursors are prepared as described in the following: U.S. Pat. Nos. 4,046,889 (1977); 4,052,511; 4,053,651; 4,113,751, 4,154,840, 4129,571 (1978), and 4,154,960 (1979) to Ondetti et al.; U.S. Pat. No. 4,626,545 (1986) to Taub; and U.S. Pat. Nos. 4,692,458 (1987) and 4,692,459 (1987) to Ryan et al., Quadro, U.S. Pat. No. 4,447,419 (1984); Haugwitz et al.; U.S. Pat. No. 4,681,886 (1987), Bush et al., U.S. Pat. No. 4,568,675 (1986), Bennion et al., U.S. Pat. No. 4,748,160 (1988), Portlock, U.S. Pat. No. 4,461,896 (1984), Hoefle et al., European Patent Application Publication No. 0 088 341 (1983), Huange et al., U.S. Pat. No. 4,585,758 (1986), European Patent application Publication No. 0 237 239, European Patent application Publication No. 0 174 162, published in 1986, European Patent application Publication No. 0 257 485, published in 1988, all of which are incorporated by reference herein.

Other suitable nitric oxide adducts include the following S-nitrosothiols: long carbon-chain lipophilic S-nitrosothiols, represented by the general formula

CH₃(CH₂)ₓ SNO,

wherein x equals 2 to 20;
S-nitroso-dithiols, represented by the general formula HS(CH₂) ₓ SNO, wherein X equals 2 to 20; and
S-nitrosothiols which possess other functional groups in addition to the thiol, and are represented by the general formula ONS(CH₂)ₓY wherein X equals 2 to 20, and Y is selected from the group consisting of fluoro, C₁-C₆ alkoxy, cyano, carboxamido, C₃-C₆ cycloalkyl, aralkoxy, C₃-C₆ alkylsulfinyl, arylthio, C₁-C₆ alkylamino, C₂-C₁₅ dialkylamino, hydroxy, carbamoyl, C₁-C₆ N-alkylcarbamoyl, C₂-C₁₅ N,N-dialkylcarbamoyl, amino, hydroxyl, carboxyl, hydrogen, nitro and aryl; wherein aryl includes benzyl, naphthyl, and anthracenyl groups. These S-nitrosothiols and the methods for preparing them are described in U.S. Patent Application No. 07/943,834, filed September 14, 1992, which is incorporated by reference herein.

Particularly preferred nitric oxide adducts include S-nitroso-proteins, such as S-nitroso-albumin, S-nitroso-tissue-type plasminogen activator, S-nitroso-hemoglobin, S-nitroso-low density lipoprotein, S-nitroso-immunoglobulin, and S-nitroso-cathepsin. Related nitric oxide adducts which are also suitable include amino acids such as tyrosine, phenylalanine, tryptophan, serine and threonine, which are nitrosoated at the oxygen, carbon, or nitrogen sites present on the amino acid. S-nitroso-proteins and nitrosated amino acids and the methods for preparing them are disclosed in U.S. Patent Application No. 07/943,835 filed September 14, 1992, and Stamler et al., Proc. Natl. Acad. Sci., USA 89:444-448 (1992), both of which are incorporated by reference herein.

Other suitable nitric oxide adducts are iron-nitrosyl compounds represented by the general formula YₓFe_{y}(NO)₂, wherein Y is an anionic species, such as thiolate, phosphate, ascorbate, proteins, or glycoaminoglycans, such as heparin sulfate and x and y are each independently 1-20. These compounds can be prepared by routine methods generally available to those in the art such as those described in Vanin et al., Studia Biophysics, 93:63-68 (1983).

Additional suitable nitric oxide adducts include thionitrates, thionitrites as well as sydnonimines, furoxans, sodium nitroprusside and organic nitrates, such as nitroglycerin, which may be obtained from generally available commercial pharmaceutical sources.

Treatment of the artificial surface with the nitric oxide adduct comprises contacting the artificial surface with the adduct so as to cause the artificial surface to be coated with the particular adduct. Coating of the artificial surface may be accomplished using the methods described in Example 1, infra, or other standard methods well known to those of ordinary skill in the art. For example, coating a surface with nitric oxide adducts can be achieved by bathing the artificial surface, either by itself or within a device, in a solution containing the nitric oxide adduct. In addition, synthetic nitric oxide adducts may be coated onto an artificial surface by a variety of chemical techniques which are well known in the art. Such techniques include attaching the adduct to a nucleophilic center, metal, epoxide, lactone, an alpha- or beta-saturated carbon chain, alkyl halide, carbonyl group, or Schiff base, by way of the free thiol.

In order to optimize the coating techniques further, standard methods may be used to determine the amount of platelet deposition on a sample of the treated artificial surface. Such methods include the use of ⁵¹Cr-labeled platelets or Indium¹¹¹-labeled platelets. Other well known techniques for evaluating platelet deposition on artificial surfaces are described in Forbes et al. (1978), and Salzman et al. (1981).

It is also contemplated that artificial surfaces will vary depending on the nature of the surface, and such characteristics as contour, crystallinity, hydrophobicity, hydrophilicity, capacity for hydrogen bonding, and flexibility of the molecular backbone and polymers. Therefore, using routine methods, one of ordinary skill will be able to customize the coating technique by adjusting such parameters as the amount of adduct, length of treatment, temperature, diluents, and storage conditions, in order to provide optimal coating of each particular type of surface.

After the device or artificial material has been coated with the nitric oxide adduct, it will be suitable for its intended use, for example, implantation as a heart valve, insertion as a catheter, or for cardiopulmonary oxygenation or hemodialysis. The coated device or artificial surface will be suitable for use in conjunction with an animal, preferably mammals, including humans.

The method of the invention provides significant advantages over current attempts to reduce platelet deposition on artificial surfaces. As demonstrated by the inventors, an artificial surface can be coated with nitric oxide adducts using simple, effective methods. The coated surfaces may be used immediately, or stored and used at a later date. In addition, by coating the surface itself, this method eliminates the need for systemic administration of anti-thrombogenic agents which are often ineffective, have serious adverse side effects, or are unsuitable for use in certain patients. Also, the inhibition of platelet deposition provided by the invention is completely and immediately reversible, a need which is especially important in patients with cardiac or vascular disease.

By preventing platelet deposition or thrombus formation on artificial surfaces, the invention is also useful in preventing serious vascular complications associated with the use of artificial surfaces. These complications occur as a result of increased platelet deposition, activation, and thrombus formation or consumption of platelets and coagulation proteins. Such complications are well known to those of ordinary skill in the medical arts and include myocardial infarction, pulmonary thromboembolism, cerebral thromboembolism, thrombophlebitis, thrombocytopenia, bleeding disorders, and any additional complication which occurs either directly or indirectly as a result of the foregoing disorders.

In another embodiment, the invention relates to a method for preventing the deposition of platelets on an artificial surface comprising contacting the artificial surface with a nitric oxide adduct in combination with at least one additional anti-thrombogenic agent. The term "anti-thrombogenic" agent refers to any compound which alters platelet function, or interferes with other mechanisms involved in blood clotting, such as fibrin formation. Examples of such compounds include, but are not limited to, heparin, warfarin, aspirin, indomethacin, dipyridamole prostacyclin, prostaglandin-E, or sulfinpyrazone.

This method for coating an artificial surface with a nitric oxide adduct in combination with another anti-thrombogenic agent will be accomplished using the methods described previously for coating an artificial surface with a nitric oxide adduct alone, and are suitable for any and all types of artificial surfaces. The appropriate coating concentration of the other anti-thrombogenic compound is determined using routine methods similar to those described previously. The coated artificial surfaces may be used in the same manner described for those artificial surfaces coated with nitric oxide adducts alone.

By coating an artificial surface with a nitric oxide adduct in combination with at least one other anti-thrombogenic agent, one will be able to not only prevent platelet deposition, which is the initial event in thrombus formation, but also to limit fibrin formation directly, by inhibiting factor VIII, and platelet granule secretion, and indirectly, by inhibiting plasminogen activator inhibitor (PAI-1) release from platelets. Thus, by coating an artificial surface with agents that both prevent platelet deposition and interfere with other platelet functions which contribute to coagulation, the invention provides a further means for preventing thrombus formation on an artificial surface.

In a further embodiment, the invention relates to the use of a nitric oxide adduct for the manufacture of a pharmaceutical preparation for preventing thrombus formation on a damaged vascular surface in an animal, comprising applying a nitric oxide adduct directly to the damaged surface. The term "damaged vascular surface" refers to any portion of the interior surface of a blood vessel in which damage to the endothelium, narrowing or stenosis of the vessel, or atherosclerotic plaque accumulation has occurred. The invention is especially suitable for use in coronary arteries, but is beneficial in other damaged arteries and also in veins including particularly those used in arterial or venous bypass replacement where they are susceptible to damage from the typically higher arterial pressures to which they are unaccustomed.

The nitric oxide adduct is applied directly to the damaged vascular surface by using an intraarterial or intravenous catheter, suitable for delivery of the adduct to the desired location. The location of damaged arterial surfaces is determined by conventional diagnostic methods, such as X-ray angiography, performed using routine and well-known methods available to those of skill within the medical arts. In addition, administration of the nitric oxide adduct using an intraarterial or intravenous catheter is performed using routine methods well known to those in the art. Typically, the preparation is delivered to the site of angioplasty through the same catheter used for the primary procedure, usually introduced to the carotid or coronary artery at the time of angioplasty balloon inflation.

The compounds of this invention can be employed in combination with conventional excipients, i. e., pharmaceutically acceptable organic or inorganic carrier substances suitable for parenteral application which do not deleteriously react with the active compounds. Suitable pharmaceutically acceptable carriers include, but are not limited to, water, salt solutions, alcohol, vegetable oils, polyethylene glycols, gelatin, lactose, amylose, magnesium stearate, talc, silicic acid, viscous paraffin, perfume oil, fatty acid monoglycerides and diglycerides, petroethral fatty acid esters, hydroxymethylcellulose, polyvinylpyrrolidone, etc. The pharmaceutical preparations can be sterilized and if desired, mixed with auxiliary agents, e.g., lubricants, preservatives, stabilizers, wetting agents, emulsifiers, salts for influencing osmotic pressure, buffers, colorings, flavoring and/or aromatic substances and the like which do not deleteriously react with the active compounds. For parenteral application, particularly suitable vehicles consist of solutions preferably oily or aqueous solutions, as well as suspensions, emulsions, or implants. Aqueous suspensions may contain substances which increase the viscosity of the suspension and include, for example, sodium carboxymethyl cellulose, sorbitol, and/or dextran. Optionally, the suspension may also contain stabilizers.

The term "therapeutically effective amount," for the purposes of the invention, refers to the amount of the nitric oxide adduct which is effective to achieve its intended purpose. While individual needs vary, determination of optimal ranges for effective amounts of each nitric oxide adduct is within the skill of the art. Generally, the dosage required to provide an effective amount of the composition, and which can be adjusted by one of ordinary skill in the art will vary, depending on the age, health physical condition, sex, weight, extent of disease of the recipient, frequency of treatment and the nature and scope of the desired effect. The preparations, which are suitable for treatment of artificial surfaces and endothelium are used in concentrations of about 500-700 mM of adduct delivered by drip infusion sterile in a physiological liquid over 2-3 minute periods in amounts of 2-3 ml per 25 kg body weight.

As demonstrated by the inventors, direct application of a nitric oxide adduct to a damaged vascular surface, coats the surface, thereby decreasing the thrombogenicity of the surface. As further demonstrated by the inventors, local application of the nitric oxide adduct to the damaged vascular surface can be accomplished at doses much lower than those required to exert a systemic effect. Thus, this method provides a significant and an unexpected advantage over the use of systemic anti-thrombogenic agents to prevent thrombus formation in damaged vessels.

### Example 1

### NO Adducts Make Artificial Surfaces Less Thrombogenic

One of the best ways to demonstrate that an artificial surface exposed to blood has been made less thrombogenic is to measure or quantitate the number of blood platelets that collect on that surface. This method requires the removal of platelets from an animal or human subject. The platelets are labeled with a radioactive material such as Indium¹¹¹, which emits gamma rays, detectable by a gamma counter placed 3 to 6 inches away from the source of radioactive platelets. The labeled platelets are either reinjected into the animal or human in vivo, or contacted with the artificial surface in vivo. Platelets will adhere to artificial surfaces or acutely damaged arterial surfaces. Thus, the number of normal platelets and radioactive platelets which stick to the surface is an indication of the thrombogenicity of the surface.

The inventors have used this methodology in experiments to demonstrate that nitric oxide adducts decrease the thrombogenicity of an artificial surface or a damaged natural arterial surface. The following experiments demonstrate that coating artificial surfaces, such as synthetic vascular graft material, with a nitric oxide adduct, decreases platelet deposition and makes the surface significantly less thrombogenic than previously used agents such as albumin alone. In addition, the experiments demonstrate that polyvinyl chloride (PVC) tubing, which is used extensively in artificial kidney and heart-lung machines, can be coated with an nitric oxide adduct such as S-nitroso-albumin, to make it less thrombogenic.

### Protection of Synthetic Vascular Grafts

First, the inventors coated dacron grafts and cardiac catheters with S-nitroso-bovine serum albumin (BSA). In three separate experiments, an identical pair of 6mm (internal diameter) knitted dacron grafts, 5 cm. in length, were prepared for surgical placement in the transected carotid arteries of three anesthetized dogs. No heparin was given. One graft was soaked in 5% BSA and the other graft was soaked in 5% BSA combined with 0.5 mM nitric oxide (producing S-nitroso-BSA) for one hour prior to insertion, and then rinsed in saline. The grafts were sutured in place with a continuous 6-0 proline suture.

### Indium-labeled platelets

Indium¹¹¹-labeled platelets are very useful in detecting platelet accumulation on vascular grafts. Therefore, Indium¹¹¹-labeled platelets were prepared according to standard methods described in Heyns AP "Method for Labeling Platelets with In¹¹¹-oxine". In: Platelet Kinetics and Imaging Vol. II Editors A.P. Heyns, P. N. Bodenhorst, M. G. Lotter CRC Press, 1985; and Sheffel V., et al., J. Nucl. Med., 20: 524-531, 1979, and injected prior to insertion of the grafts. Following graft insertion, the dogs were observed for two hours, then both grafts were removed, rinsed, and weighed. The grafts were then placed in a Nal gamma well counter and counted for four minutes.

The three grafts coated with BSA alone had an average of 654,000 +/-89,000 counts/4 minutes. In contrast, the three grafts coated with S-nitroso-BSA had an average of 278,000 +/57,000 counts/4 minutes (P < .005). The average percent increase in weight for the three grafts due to thrombus formation on the luminal surface with BSA alone, was 410% +/-97%, while the percent increase in weight for the three grafts incubated with nitroso-BSA was 196 % +/- 71 % (P < 0. 005).

These data show that during exposure of the graft to circulating blood over a period of two hours, there was considerably less platelet deposition and clotting on the synthetic grafts treated with S-nitroso-BSA. Thus the results demonstrate that S-nitroso-BSA coating of synthetic vascular grafts provides protection against early platelet deposition.

In addition, three pairs of 5 FR USC1 catheters were studied. One catheter was soaked in 5% BSA, while the other catheter was soaked in a mixture of S-nitroso BSA for one hour. The catheters were rinsed with saline and one each was inserted into the right or left femoral arteries of the dogs described above, and left for two hours. Each catheter was flushed with normal saline every one-half hour, but no heparin was given. The catheters were then removed and rinsed with saline. Equal lengths of the catheters were cut from the distal ends and each one was placed in a Nal gamma counter and the radioactivity was counted for four minutes.

The counts for the three catheters coated with BSA alone had an average count of 9,000 ± 1, 100. In contrast, the three catheters coated with 5 % BSA + 0.5 Mm nitric oxide had only 2,850 ± 800 counts. Thus, there were significantly fewer platelets deposited on the catheters coated with S-nitroso-BSA, than those coated with BSA alone. These experiments demonstrate that synthetic vascular grafts coated with S-nitroso-BSA and immediately implanted, are significantly less thrombogenic than grafts coated with BSA alone.

The inventors conducted an additional experiment to investigate whether S-nitroso-BSA can be used to coat a surface such as polyvinyl chloride (PVC), and in addition, whether such surfaces can be treated at one time, and used at a later time. In this experiment, three pieces of PVC, 3 mm in internal diameter and 2 cm. in length were soaked in BSA for 4 hours, allowed to dry, and placed in a dark place. Three identical pieces of PVC tubing were soaked in an S-nitroso-BSA solution for 4 hours, dried, and also placed in the dark. The lengths of PVC tubing were kept in the dark to minimize potential inactivation of the nitric oxide-donating compounds caused by exposure to light.

Three days after coating, a pair of PVC tubing pieces, one coated with BSA, and one coated with S-nitroso-BSA, were placed as a shunt in each of the two femoral arteries of a dog. The dog was injected with Indium" 'labeled platelets as previously described. Two hours after the PVC shunts were placed in the circulation with radioactive platelets, they were removed and placed in the Nal gamma counter.

The counts on the BSA coated shunt were 200,870/4 minutes, whereas on the S-nitroso-BSA coated graft, the counts were only 97,510/4 minutes. Thus, the shunts coated with S-nitroso-BSA have significantly fewer platelets deposited on its internal surface than the one coated with nitroso-BSA.

### Example 2

### Na Nitroprusside Coated Damaged Arterial Surfaces Are Less Thrombogenic.

The following experiments demonstrate that nitric oxide-donating compounds, such as sodium nitroprusside and S-nitroso-BSA, can be applied directly to damaged arterial or venous surfaces (blood vessels) to inhibit platelet deposition and thrombus formation.

The inventors developed an animal model which allows them to mimic a patient with narrowing of the coronary or other arteries and arterial damage caused by atherosclerosis or after angioplasty, atherectomy or other procedure. The model uses anesthetized dogs with open chest and exposed heart. Briefly, an electromagnetic flow probe is placed on the coronary artery to continuously measure blood flow through the artery. Then the arterial wall is damaged(intima and media) by clamping the artery several times with a surgical clamp. In the area of arterial damage, a plastic encircling cylinder is placed around the outside of the coronary artery to produce a 70% narrowing or reduction in the lumen gradually diameter. This mimics atherosclerotic narrowing of arteries in patients. Platelet-mediated thrombi periodically form in the stenosed lumen, gradually cutting off the coronary blood flow. Subsequently, the thrombi embolize distally and blood flow is restored. This process, which occurs periodically, produces cyclical reductions in flow, hereinafter referred to as "cyclic flow reductions" (CFRs). If no action is taken to prevent platelet interaction with the damaged arterial wall, these CFRs will continue to occur for many hours.

The inventors have determined that CFRs represent an interaction between platelets and the clotting system, and damaged endothelial cells in narrowed or stenosed arterial walls. In addition, CFRs occur in human arteries which are narrowed by atherosclerosis, and the resulting periodic clot formation can cause chest pain or leg pain in patients with atherosclerotic narrowing of coronary or leg arteries. Finally, the CFRs due to platelet-mediated clotting can be exacerbated by further damage to the arterial wall.

During the course of this study it was observed that when arterial wall was damaged initially by clamping the artery with a surgical clamp, platelet thrombi formed, and CFRs were produced. As a result of this observation, the following experiments were conducted to determine if direct infusion of an NO donor such as sodium nitroprusside can make a damaged arterial wall less thrombogenic.

The following experiments demonstrate that nitric oxide-donating compounds, such as sodium nitroprusside and S-nitroso-BSA can be applied directly to damaged arterial surfaces (blood vessels) to inhibit platelet deposition and thrombus formation.

In five anesthetized dogs, both carotid arteries were exposed. Two 3 FR USC1 catheters were prepared for arterial implantation. One catheter was soaked in a 5% BSA solution for 12 hours, while the other was soaked in a 5% BSA solution which also contained 1 mg/ml of sodium nitroprusside. One each of the two coated catheters was placed randomly in the right or left carotid artery of the dog through a small incision sealed with a 6-0 proline suture. The catheters were advanced for 5 cm into the arterial lumen. The dogs were not given any heparin. The catheters were removed 6-8 hours later and examined for clotting on the catheter wall and at the site where the catheter entered the carotid wall. There was considerably more clotting on the BSA-coated catheter compared to the catheter coated with BSA plus sodium nitroprusside.

In five open-chested anesthetized dogs, the coronary artery was dissected out and instrumented for measuring CFRs as previously described. The inventors observed that intravenous infusion of sodium nitroprusside directly into the artery (at a dose of between 4 and 10 µg/kg/min. for up to 30 minutes) resulted in a decrease in vivo platelet activity and CFRs were abolished. In addition, the circulating nitroprusside appeared to coat the damaged arterial wall, thus making it less thrombogenic. The CFRs were observed to continue until the sodium nitroprusside infusion had been given for 15 minutes. Then, the CFRs ceased, which suggests that the platelets were no longer adhering to the arterial wall. The sodium nitroprusside intravenous infusion was then stopped. The direct in vivo inhibition of circulating platelets normally stops within 10-15 minutes. However, after the in vivo inhibition of the platelets by the presence of circulating sodium nitroprusside was gone, the CFRs did not return. This indicates that the previously circulating sodium nitroprusside left a protective coating on the previously damaged arterial surface. The inventors have termed this protective coating process "passivation."

The inventors then showed that if one gently rolls the artery between the fingers, the CFRs return immediately. This suggests that the protective coating provided by sodium nitroprusside, has been removed from the internal surface of the previously damaged artery, thus, allowing platelets to resume interaction with the unprotected arterial wall and produce CFRs. In order to demonstrate that this was a local phenomenon affecting the damaged artery, and not due to a systemic effect inhibiting all the circulating platelets, the following experiments were performed.

Open-chest anesthetized dogs were studied. In the dog, and also in humans, the two major branches of the main left coronary artery which are approximately equal in size, are called the left circumflex (circ) and the left anterior descending (LAD), coronary arteries. In the experiments, both branches were instrumented with a flow measuring device, were given equal arterial wall damage (endothelial and medial damage), and had encircling plastic cylinders placed on them to produce equal amounts of narrowing or stenosis.

Following the induction of damage in both coronary arterial branches, CFRs were observed in both the LAD branch and the circumflex branches of the left coronary artery, indicating that the circulating platelets were adhering to both the narrowed part of the damaged circumflex artery and also to the damaged LAD artery. Sodium nitroprusside (10 mg/kg) was then infused directly into the circumflex coronary artery over 30 seconds. Following the infusion, the CFRs in the circ disappeared while they continued in the LAD coronary artery. This demonstrates that the sodium nitroprusside had a local protective effect on the damaged circ, and that the dose of sodium nitroprusside was not high enough to affect circulating platelets or, after recirculation dilution, to protect the damaged LAD wall.

CFRs due to platelets adhering and aggregating on the damaged arterial walls were observed in both arteries, each independent of the other. Therefore, by injecting the sodium nitroprusside into the circumflex branch, the inventors were able to coat this damaged artery directly. In addition, the circulating concentration of sodium nitroprusside remaining after local infusion appears to be too low to have a systemic effect on platelets. Thus, the inventors demonstrated that the protective effect exerted by localized application of sodium nitroprusside is a local effect, and can be applied directly to protect particular segments of a damaged artery.

Experiments identical to those described above were repeated using a nitric oxide-bovine serum albumin adduct (BSA-NO) (with approximately 0.5 mM NO concentration) given selectively into the circumflex coronary artery. The inventors show that using BSA-NO as the NO adduct provides better passivation and the effect lasts longer. When the protective BSA-NO coating has been on the damaged arterial wall for 4 to 5 hours, the BSA can be recharged with new NO molecules by infusing sodium nitroprusside intravenously (5-10 µg/kg for 20 minutes) or directly into the coronary artery (10 mg/kg for 30 seconds).

## Claims

1. A method for preventing the deposition of platelets or thrombus formation on an artificial surface which comes into contact with blood comprising contacting said artificial surface with a nitric oxide adduct.

2. The method of claim 1, wherein said artificial surface comprises a medical device.

3. The method of claim 2, wherein said medical device is a catheter, prosthetic heart valve, artificial cardiopulmonary support apparatus, synthetic vessel graft, hemodialysis membrane, blood conduit tubing, blood storage bag, arteriovenous shunt or artificial heart.

4. The method of claim 1, wherein said nitric oxide adduct comprises a sodium nitroprusside, a S-nitrosothiol, a S-nitrosodithiol, a thionitrate, a thionitrite, a nitrosated amino acid, an organic nitrate, a iron-nitrosyl compound, a sydnonimine, a furoxan or a S-nitroso-protein.

5. The method of claim 4, wherein said S-nitrosothiol is a long carbon chain lipophilic nitrosothiol, S-nitroso-N-acetylcysteine, S-nitroso-captopril, S-nitroso-homocysteine, S-nitroso-cysteine, S-nitrosoglutathione or S-nitrosopenicillamine.

6. The method of claim 4, wherein said organic nitrate is nitroglycerin.

7. The method of claim 4, wherein said S-nitroso-protein is a S-nitroso-albumin, a S-nitroso-tissue-type plasminogen activator, a S-nitroso-hemoglobin, a S-nitroso-lipoprotein, a S-nitroso-immunoglobulin or a a S-nitroso-cathepsin.

8. The method of claim 7, wherein said S-nitroso-albumin is S-nitroso-bovine serum albumin.

9. A method according to any one of claims 1 to 8 wherein said nitric oxide adduct is used in combination with an anti-thrombogenic compound.

10. The method of claim 9, wherein said anti-thrombogenic compound comprises heparin, hirudin and its analogues, warfarin, aspirin, indomethacin, dipyridamole, prostacyclin, prostaglandin-E or sulfinpyrazone, phenothiazine, RGD peptide and RGD peptide mimetics, agents that block platelet glycoprotein IIb-IIIa receptors, ticlopidine or clopidogrel.

11. Use of a therapeutically effective amount of a nitric oxide adduct for the manufacture of a pharmaceutical preparation for preventing thrombus formation on a damaged vessel surface.

12. Use of a therapeutically effective amount of a nitric oxide adduct for the manufacture of a pharmaceutical preparation for treating or preventing a myocardial infarction, thrombophlebitis, thrombocytopenia or a bleeding disorder.

13. Use of a therapeutically effective amount of a nitric oxide adduct for the manufacture of a pharmaceutical preparation for inhibiting cyclic reductions in blood flow through a portion of a vascular lumen, wherein at least a portion of the vascular lumen has a damaged vascular surface.

14. Use according to any one of claims 11 to 13, wherein said nitric oxide adduct comprises sodium nitroprusside, a S-nitrosothiol, a S-nitrosodithiol, a thionitrate, a thionitrite, a nitrosated amino acid, an organic nitrate, a iron-nitrosyl compound, a sydnonimine, a furoxan or a S-nitroso-protein.

15. Use according to claim 14, wherein said S-nitrosothiol is a long carbon chain lipophilic nitrosothiol, S-nitroso-N-acetylcysteine, S-nitroso-captopril, S-nitroso-homocysteine, S-nitroso-cysteine, S-nitrosoglutathione or S-nitrosopenicillamine.

16. Use according to claim 14, wherein said organic nitrate is nitroglycerin.

17. Use according to claim 14, wherein said nitroso-protein is a S-nitroso-albumin, a S-nitroso-tissue-type plasminogen activator, a S-nitroso-hemoglobin, a S-nitroso-Iow density lipoprotein, a S-nitroso-immunoglobulin or a S-nitroso-cathepsin.

18. Use according to claim 17, wherein said S-nitroso-albumin is S-nitroso-bovine serum albumin.

19. Use according to any one of claims 11 to 18, wherein said nitric oxide adduct is used in combination with an anti-thrombogenic compound.

20. Use according to claim 19, wherein said anti-thrombogenic compound comprises heparin, hirudin and its analogues, warfarin, aspirin indomethacin, dipyridamole, prostacyclin, prostaglandin-E or sulfinpyrazone, phenothiazine, RGD peptides and RGD peptide mimetics, agents that block platelet glycoprotein IIb-IIIa receptors, ticlopidine or clopidogrel.

21. Use according to claim 11, wherein the damaged vascular surface is the interior surface of a blood vessel in which damage to the endothelium, narrowing, stenosis of the vessel or atherosclerotic plaque accumulation has occurred.

22. Use according to claim 11, wherein the nitric oxide adduct is delivered by an intraarterial catheter or an intravenous catheter.

23. An artificial surface coated with a nitric oxide adduct.

24. An artificial surface according to claim 23, wherein said nitric oxide adduct comprises a sodium nitroprusside, a S-nitrosothiol, a S-nitrosodithiol, a thionitrate, a thionitrite, a nitrosated amino acid, an organic nitrate, a iron-nitrosyl compound, a sydnonimine, a furoxan or a S-nitroso-protein.

25. An artificial surface according to claim 24, wherein said S-nitrosothiol is a long carbon chain lipophilic nitrosothiol, S-nitroso-N-acetylcysteine, S-nitroso-captopril, S-nitroso-homocysteine, S-nitroso-cysteine, S-nitrosoglutathione or S-nitrosopenicillamine.

26. An artificial surface according to claim 24, wherein said organic nitrate is nitroglycerin.

27. An artificial surface according to claim 24, wherein said S-nitroso-protein is a S-nitroso-albumin, a S-nitroso-tissue-type plasminogen activator, a S-nitroso-hemoglobin, a S-nitroso-lipoprotein, a S-nitroso-immunoglobulin or a a S-nitroso-cathepsin.

28. An artificial surface according to claim 27, wherein said S-nitroso-albumin is S-nitroso-bovine serum albumin.

29. An artificial surface according to any one of claims 23 bis 28, wherein said nitric oxide adduct is used in combination with an anti-thrombogenic compound.

30. An artificial surface according to claim 29, wherein said anti-thrombogenic compound comprises heparin, hirudin and its analogues, warfarin, aspirin, indomethacin, dipyridamole, prostacyclin, prostaglandin-E or sulfinpyrazone, phenothiazine, RGD peptide and RGD peptide mimetics, agents that block platelet glycoprotein IIb-IIIa receptors, ticlopidine or clopidogrel.

## Patentansprüche

1. Verfahren zum Vermeiden der Ablagerung von Blutplättchen oder einer Thrombusbildung auf einer künstlichen Oberfläche, die mit Blut in Kontakt kommt, umfassend das Inkontaktbringen der künstlichen Oberfläche mit einem Stickoxidaddukt.

2. Verfahren gemäß Anspruch 1, wobei die künstliche Oberfläche eine medizinische Vorrichtung umfasst.

3. Verfahren gemäß Anspruch 2, wobei die medizinische Vorrichtung ein Katheter, eine Herzklappenprothese, eine künstliche Herz-Lungen-Unterstützungsapparatur, ein synthetisches Gefäßtransplantat, eine Hämodialysemembran, ein Blutleitungsschlauch, Blutaufbewahrungsbeutel, arteriovenöser Shunt oder künstliches Herz ist.

4. Verfahren gemäß Anspruch 1, wobei das Stickoxidaddukt ein Natriumnitroprussid, ein S-Nitrosothiol, ein S-Nitrosodithiol, ein Thionitrat, ein Thionitrit, eine nitrosierte Aminosäure, ein organisches Nitrat, eine Eisennitrosylverbindung, ein Sydnonimin, ein Furoxan oder ein S-Nitroso-Protein umfasst.

5. Verfahren gemäß Anspruch 4, wobei das S-Nitrosothiol ein lipophiles Nitrosothiol mit langer Kohlenstoffkette, S-Nitroso-N-acetylcystein, S-Nitrosocaptopril, S-Nitrosohomocystein, S-Nitrosocystein, S-Nitrosoglutathion oder S-Nitrosopenicillinamin ist.

6. Verfahren gemäß Anspruch 4, wobei das organische Nitrat Nitroglycerin ist.

7. Verfahren gemäß Anspruch 4, wobei das S-Nitrosoprotein ein S-Nitrosoalbumin, ein S-Nitroso-gewebetypischer Plasminogenaktivator, ein S-Nitrosohämoglobin, ein S-Nitrosolipoprotein, ein S-Nitrosoimmunglobulin oder ein S-Nitrosocathepsin ist.

8. Verfahren gemäß Anspruch 7, wobei das S-Nitrosoalbumin S-Nitroso-Rinderserumalbumin ist.

9. Verfahren gemäß einem der Ansprüche 1 bis 8, wobei das Stickoxidaddukt in Kombination mit einer antithrombogenen Verbindung verwendet wird.

10. Verfahren gemäß Anspruch 9, wobei die antithrombogene Verbindung Heparin, Hirudin und deren Analoga, Warfarin, Aspirin, Indomethacin, Dipyridamol, Prostacyclin, Prostaglandin-E oder Sulfinpyrazon, Phenothiazin, RGD-Peptid und RGD-Peptid-Mimetika, Wirkstoffe, die die Thrombozyten-Glycoprotein IIb-IIIa-Rezeptoren blockieren, Ticlopidin oder Clopidogrel umfasst.

11. Verwendung einer therapeutisch wirksamen Menge eines Stickoxidaddukts zur Herstellung eines pharmazeutischen Präparats zum Vermeiden von Thrombusbildung an einer beschädigten Gefäßoberfläche.

12. Verwendung einer therapeutisch wirksamen Menge eines Stickoxidaddukts zur Herstellung eines pharmazeutischen Präparats zum Behandeln oder Vermeiden eines Herzinfarktes, akuter Thrombophlebitis, Thrombozytopenie oder Blutungsstörung.

13. Verwendung einer therapeutisch wirksamen Menge eines Stickoxidaddukts zur Herstellung eines pharmazeutischen Präparates zur Hemmung von periodischen Verringerungen des Blutflusses durch einen Teil eines Gefäßlumens, wobei wenigstens ein Teil des Gefäßlumens eine beschädigte Oberfläche hat.

14. Verwendung gemäß einem der Ansprüche 11 bis 13, wobei das Stickoxidaddukt ein Natriumnitroprussid, ein S-Nitrosothiol, ein S-Nitrosodithiol, ein Thionitrat, ein Thionitrit, eine nitrosierte Aminosäure, ein organisches Nitrat, eine Eisennitrosylverbindung, ein Sydnonimin, ein Furoxan oder ein S-Nitrosoprotein umfasst.

15. Verwendung gemäß Anspruch 14, wobei das S-Nitrosothiol ein lipophiles Nitrosothiol mit langer Kohlenstoffkette, S-Nitroso-N-acetylcystein, S-Nitrosocaptopril, S-Nitrosohomocystein, S-Nitrosocystein, S-Nitrosoglutathion oder S-Nitrosopenicillinamin ist.

16. Verwendung gemäß Anspruch 14, wobei das organische Nitrat Nitroglycerin ist.

17. Verwendung gemäß Anspruch 14, wobei das Nitrosoprotein ein S-Nitrosoalbumin, ein S-Nitroso gewebetypischer Plasminogenaktivator, ein S-Nitrosohämoglobin, ein S-Nitrosolipoprotein von niedriger Dichte, ein S-Nitrosoimmunglobulin oder S-Nitrosocathepsin ist.

18. Verwendung gemäß Anspruch 17, wobei das S-Nitrosoalbumin S-Nitroso-Rinderserumalbumin ist.

19. Verwendung gemäß einem der Ansprüche 11 bis 18, wobei das Stickoxidaddukt in Kombination mit einer antithrombogenen Verbindung verwendet wird.

20. Verwendung gemäß Anspruch 19, wobei die antithrombogene Verbindung Heparin, Hirudin und deren Analoga, Warfarin, Aspirin, Indomethacin, Dipyridamol, Prostacyclin, Prostaglandin-E oder Sulfinpyrazon, Phenothiazin, RGD-Peptide und RGD-Peptid-Mimetika, Wirkstoffe, die die Thrombozyten-Glycoprotein IIb-IIIa-Rezeptoren blockieren, Ticlopidin oder Clopidogrel umfasst.

21. Verwendung gemäß Anspruch 11, wobei die beschädigte Gefäßoberfläche die Innenseite eines Blutgefäßes ist, wobei ein Schaden an dem Endothel, eine Verengung, eine Stenose des Gefäßes oder eine arteriosklerotische Plaqueansammlung aufgetreten ist.

22. Verwendung gemäß Anspruch 11, wobei das Stickoxidaddukt durch einen intraarteriellen Katheter oder einen intravenösen Katheter abgegeben wird.

23. Künstliche Oberfläche, die mit einem Stickoxidaddukt überzogen ist.

24. Künstliche Oberfläche gemäß Anspruch 23, wobei das Stickoxidaddukt ein Natriumnitroprussid, ein S-Nitrosothiol, ein S-Nitrosodithiol, ein Thionitrat, ein Thionitrit, eine nitrosierte Aminosäure, ein organisches Nitrat, eine Eisennitrosylverbindung, ein Sydnonimin, ein Furoxan oder ein S-Nitrosoprotein umfasst.

25. Künstliche Oberfläche gemäß Anspruch 24, wobei das S-Nitrosothiol ein lipophiles Nitrosothiol mit langer Kohlenstoffkette, S-Nitroso-N-acetylcystein, S-Nitrosocaptopril, S-Nitrosohomocystein, S-Nitrosocystein, S-Nitrosoglutathion oder S-Nitrosopenicillinamin ist.

26. Künstliche Oberfläche gemäß Anspruch 24, wobei das organische Nitrat Nitroglycerin ist.

27. Künstliche Oberfläche gemäß Anspruch 24, wobei das S-Nitrosoprotein ein S-Nitrosoalbumin, ein S-Nitroso-gewebetypischer Plasminogenaktivator, ein S-Nitrosohämoglobin, ein S-Nitrosolipoprotein, ein S-Nitrosoimmunglobulin oder ein S-Nitrosocathepsin ist.

28. Künstliche Oberfläche gemäß Anspruch 27, wobei das S-Nitrosoalbumin S-Nitroso-Rinderserumalbumin ist.

29. Künstliche Oberfläche gemäß einem der Ansprüche 23 bis 28, wobei das Stickoxidaddukt in Kombination mit einer antithrombogenen Verbindung verwendet wird.

30. Künstliche Oberfläche gemäß Anspruch 29, wobei die antithrombogene Verbindung Heparin, Hirudin und deren Analoga, Warfarin, Aspirin, Indomethacin, Dipyridamol, Prostacyclin, Prostaglandin-E oder Sulfinpyrazon, Phenothiazin, RGD-Peptid und RGD-Peptid-Mimetika, Wirkstoffe, die die Thrombozyten-Glycoprotein IIb-IIIa-Rezeptoren blockieren, Ticlopidin oder Clopidogrel umfasst.

## Revendications

1. Une méthode de prévention du dépôt de plaquettes ou de formation de thrombus sur une surface artificielle venant au contact du sang, méthode consistant à amener en contact ladite surface artificielle avec un produit d'addition de l'oxyde nitrique.

2. La méthode de la revendication 1, dans laquelle ladite surface artificielle consiste en un dispositif médical.

3. La méthode de la revendication 2, dans laquelle ledit dispositif médical est un cathéter, une valvule cardiaque prothétique, un appareil de support cardio-pulmonaire, une greffe de vaisseaux synthétiques, une membrane d'hémodialyse, une tubulure de conduite de sang, un sac de stockage de sang, une déviation artéro-veineuse ou un coeur artificiel.

4. La méthode de la revendication 1, dans laquelle ledit produit d'addition d'oxyde nitrique consiste en un sel de sodium de l'acide nitroprussique, un S-nitrosothiol, un S-nitrosodithiol, un thionitrate, un thionitrite, un aminoacide nitrosé, un nitrate organique, un composé fer-nitrosyle, une sydnonimine, un furoxane ou une S-nitrosoprotéine.

5. La méthode de la revendication 4, dans laquelle ledit S-nitrosothiol est un nitrosothiol lipophile à longue chaîne carbonée, de la S-nitroso-N-acétylcystéine, du S-nitrosocaptopryl, de la S-nitroso-homocystéine, de la S-nitrosocystéine, de la S-nitrosoglutathione, ou de la S-nitrosopénicillamine.

6. La méthode de la revendication 4, dans laquelle ledit nitrate organique est la nitroglycérine.

7. La méthode de la revendication 4,dans laquelle ladite S-nitrosoprotéine est une S-nitroso-albumine, un activateur plasminogène du type tissu S-nitroso, une S-nitroso-hémoglobine, une S-nitroso-lipoprotéine, une S-nitrizo-immunoglobuline ou une S-nitroso-cathepsine.

8. La méthode de la revendication 7, dans laquelle ladite S-nitroso-albumine est de l'albumine de sérum bovin S-nitrozé.

9. Une méthode selon l'une quelconque des revendications 1 à 8, dans laquelle ledit produit d'addition de l'acide nitrique est utilisé en combinaison avec un composé anti-thrombogénique.

10. La méthode de la revendication 9, dans laquelle ledit composé anti-thrombogénique consiste en héparine, hirudine et ses homologues, warfarine, aspirine, indométhacine, dipyridamole, prostacycline, prostaglandine-E ou sulfine-pyrazone, phénothiazine, peptide RGD et produits mimétiques du peptide RGD, agents qui bloquent les récepteurs IIb-IIIo de la glucoprotéine des plaquettes, ticlopidine ou clopidogrel.

11. Utilisation d'une quantité thérapeutiquement efficace d'un produit d'addition de l'acide nitrique en vue de fabrication d'une préparation pharmaceutique destinée à la prévention de la formation de thrombus sur la surface endommagée d'un vaisseau.

12. Utilisation d'une quantité thérapeutiquement efficace d'un produit d'addition d'oxyde nitrique en vue de la fabrication d'une préparation pharmaceutique destinée au traitement ou à la prévention d'un infarctus du myocarde, d'une thrombophlébite, d'une thrombocytopenie ou d'un désordre du saignement.

13. Utilisation d'une quantité thérapeutiquement efficace d'un produit d'addition de l'acide nitrique en vue de la fabrication d'une préparation pharmaceutique destinée à inhiber les réductions cycliques du débit sanguin sur une portion d'un orifice vasculaire, dans laquelle au moins une portion de l'orifice vasculaire présente une surface vasculaire endommagée.

14. Utilisation selon l'une quelconque des revendications 11 à 13, dans laquelle ledit produit d'addition de l'oxyde nitrique consiste en sel de sodium de l'acide nitroprussique, un S-nitrosothiol, un S-nitrosodithiol, un thionitrate, un thionitrite, un acide aminé nitrosé, un nitrate organique, un composé fer-nitrozyl, une sydnonimine, un furoxane ou une protéine S-nitrozée.

15. Utilisation selon la revendication 14, dans laquelle le di-S-nitrosothiol est un nitrosothiol lipophile à longue chaîne carbonée, de la S-nitroso-N-acétylcystéine, du S-nitroso-captopril, la S-nitroso-homocystéine, la S-nitroso-cystéine, la S-nitrosoglutathione ou la S-nitrosopénicillamine.

16. Utilisation selon la revendication 14, dans laquelle ledit nitrate organique est la nitroglycérine.

17. Utilisation selon la revendication 14, dans laquelle ladite nitroso-protéine est une S-nitroso-albumine, un activateur plasminogène du type tissu S-nitroso, une S-nitroso-hémoglobine, une lipoprotéine à basse densité, une S-nitrizo-immunoglobuline ou une S-nitroso-cathepsine.

18. Utilisation selon la revendication 17, dans laquelle ladite S-nitroso-albumine est une albumine de sérum bovin S-nitrozée.

19. Utilisation selon l'une quelconque des revendications 11 à 18, dans laquelle ledit produit d'addition de l'oxyde nitrique est utilisé en combinaison avec un composé anti-thrombogénique.

20. Utilisation selon la revendication 19, dans laquelle ledit composé anti-thrombogénique consiste en héparine, hirudine et ses analogues, warfarine, aspirine, indométhacine, dipyridamole, prostacycline, prostaglandine-E ou sulfine-pyrazone, phénothiazine, peptides RGD et agents mimétiques du peptide RGD, agents qui bloquent les récepteurs IIb-IIIa de la glucoprotéine des plaquettes, ticlopidine ou clopidogrel.

21. Utilisation selon la revendication 11, dans laquelle la surface vasculaire endommagée est la surface intérieure d'un vaisseau sanguin dans laquelle se sont produits des dommages à l'endothélium, un rétrécissement, une sténose du vaisseau ou une accumulation athérosclérotique de plaques.

22. Utilisation selon la revendication 11, dans laquelle le produit d'addition de l'acide nitrique est délivré par un cathéter intra-artériel ou un cathéter intraveineux.

23. Une surface artificielle recouverte par un produit d'addition de l'oxyde nitrique.

24. Une surface artificielle selon la revendication 23, dans laquelle ledit produit d'addition de l'oxyde nitrique consiste en un sel de sodium de l'acide nitroprussique, un S-nitrosothiol, un S-nitroso-dithiol, un thionitrate, un thionitrite, un amino-acide nitrosé, un nitrate organique, un composé fer-nitrosile, une sydnonimine, un furoxane ou une protéine S-nitrozée.

25. Une surface artificielle selon la revendication 24, dans laquelle ledit S-nitrosothiol est un nitrosothiol lipophile à longue chaîne carbonée de la S-nitroso-N-acétylcystéine, du S-nitrosocaptopril, de la S-nitroso-homocystéine, de la S-nitroso-cystéine, de la S-nitrosoglutathione ou de la S-nitrosopénicillamine.

26. Une surface artificielle selon la revendication 24, dans laquelle ledit nitrate organique est la nitroglycérine.

27. Une surface artificielle selon la revendication 24, dans laquelle ladite S-nitroso-protéine est une S-nitroso-albumine, un activateur du plasminogène du type tissu S-nitrozé, une S-nitroso-hémoglobine, une S-nitrosoprotéine, une S-nitroso-immunoglobuline ou une S-nitroso-cathepsine.

28. Une surface artificielle selon la revendication 27, dans laquelle ladite S-nitroso-albumine est de l'albumine de sérum bovin S-nitrozée.

29. Une surface artificielle selon l'une quelconque des revendications 23 à 28, dans laquelle ledit produit d'addition de l'oxyde nitrique est utilisé en combinaison avec un composé anti-thrombogénique.

30. Une surface artificielle selon la revendication 29, dans laquelle ledit composé anti-thrombogénique consiste en héparine, hirudine et ses analogues, warfarine, aspirine, indométhacine, dipyridamole, prostacycline, prostaglandine-E ou sulfine pyrazone, phenothiazine, peptides RGD et produits mimétiques du peptide RGD, agents qui bloquent les récepteurs Iib-IIIa de la glycoprotéine des plaquettes, ticlopidine ou clopidogrel.
